# EUROPEAN PATENT SPECIFICATION

(11) **EP 1 773 143 B1**
(45) Date of publication and mention of the grant of the patent: **13.10.2010**
(21) Application number: 05857541.6
(22) Date of filing: 20.07.2005
(51) Int. Cl.: A41D 13/002

(54) **GAS DISTRIBUTION GARMENT**
GASVERTEILUNGKLEIDUNG
VÊTEMENT À DISTRIBUTION DE GAZ

(30) Priority: 06.08.2004 US 913975
(43) Date of publication of application: 18.04.2007
(73) Proprietor: Gore Enterprise Holdings, Inc., Newark, DE 19714-9206 (US)
(72) Inventor: DACEY, Paul, Newark, DE 19713 (US); FARNWORTH, Brian, Burnaby, BC V5A 4Y8 (CA)
(74) Representative: Shanks, Andrew
(86) International application number: PCT/US2005/025851
(87) International publication number: WO 2006/085998

(56) References cited:
- EP-A- 0 490 347
- EP-A- 0 948 908
- EP-A- 1 101 429
- DE-C- 692 245
- US-A- 5 243 706
- US-A- 5 564 124
- US-A- 5 970 519
- US-A1- 2004 083 526

## Description

### FIELD OF THE INVENTION

The present invention relates to a personal gas distribution garment, preferably a ventilated cooling garment. One embodiment is directed to a ventilated cooling garment for use by a wearer who is clad in a sealed overall suit and breathing system which is designed to protect the wearer from harmful chemical, biological or other environmental hazards. It is also a function of the ventilated cooling garment of the present invention that it may be adapted to use filtered ambient air as the ventilating cooling medium. Further desirable attributes of the garment are high cooling power, low weight, low bulk, good flexibility, and high water vapour permeability, all of which contribute to the comfort of the wearer.

### BACKGROUND OF THE INVENTION

It is well known that subjecting a person to prolonged periods of inadequate heat dissipation leads to an increase in body temperature (heat stress), indicated by undesirable effects such as discomfort, increased fatigue, decreased physical and intellectual performance and, in extreme cases, death. Body core temperatures in excess of 38 °C will, for example, lead to impaired decision making and increased reaction times whereas core temperatures in excess of 40 °C can cause physiological damage and fatalities. Increased body temperature can result from accumulation of heat from external sources, metabolic processes due to exertion, or a combination of both. Personnel such as fire-crews, "hazmat" operatives such as those working on toxic or generally hazardous cleanup operations, and chemical plant operatives handling hazardous products are potential victims of such heat stress. Such personnel have usually to wear virtually totally sealed garments which severely inhibit cooling effects that would naturally occur due to ambient air flow over the persons skin and clothing.

One possible measure to prevent the onset of heat-stress is to blow a cooling gas, usually air, optionally cooled, over the subject's body, which results in cooling of the subject by a combination of convective and evaporative cooling. Studies of heat stress effects have shown that, to minimise such effects, the average desirable amount of cooling supplied to a subject undergoing moderate exertion is a minimum of 100 watts over the area of the torso. (Ref.:"Techniques for Estimating Ventilation Requirements for Personal Air-cooling Systems", J. W. Kaufman, Naval Air Warfare Center report NAWCADPAX-99-92-TR.)

Various approaches have been proposed to achieve "air-cooling" of subjects. For example, a system disclosed in U.S. Patent No. 5,243,706 to Frim et al. is one such approach. The construction of the garment disclosed in this reference comprises an air-impermeable layer and an air distribution layer attached together with a corrugated mesh spacer layer in between. A further mesh spacer layer is positioned between the air-permeable layer and the body of the wearer. Cooling air is fed into the space between the air- permeable and air-impermeable layers, exits the air permeable layer, and is distributed over the body of the wearer. Given the multi-layer construction of the garment and the inclusion of the corrugated spacer layer the flexibility, fit and comfort of the garment would be severely compromised and would be unlikely to meet the desirability criteria defined *supra.* Also, the relatively high resistance of the mesh fabrics to the flow of air necessitates a high pressure air source not readily available in a portable (or non-tethered) system U.S Patent No. 5,243,706 discloses the preamble of claim 1 and the preamble of claim 41.

U.S. Patent No. 5,564,124 to Elsherif et al. discloses a personal ventilation apparatus which comprises a garment incorporating areas of air permeable material, such as open cell foam, to direct air to selected areas of the body. The system also comprises a battery powered blower unit which, optionally, includes thermoelectric heating or cooling devices or filters. Given the small areas over which the cooling air is vented relative to the total area of the torso, the cooling power of the garment disclosed in this reference is likely to be severely limited and not meet the cooling criteria previously defined.

U.S. Patent No. 5,970,519 to Weber discloses a cooling garment for medical personnel which comprises a simple two ply construction of an air impermeable layer and an air permeable layer, each having minimal thickness, defining a cavity into which air is blown. The cavity has no spacers, or intermediate material or structures except in the shoulder regions to prevent the collapse of the garment in that area when the garment is worn under a heavy apron such as a radiological shield. One distinct shortcoming of such a system is the absence of any intermediate layer to control airflow within the cavity resulting in uneven air distribution. A further shortcoming is the lack of a means for controlling air distribution between the inner air permeable layer and the body of the wearer. The absence of such mechanisms may cause excessive cooling of some areas of the wearer's body, especially next to the air inlet port, while not supplying sufficient cooling in other areas. It is an objective of the present invention to overcome the shortcomings of the systems described above.

### SUMMARY OF THE INVENTION

The present invention is directed to a gas distribution garment system which can be used with sealed garments such as are used in hazardous or toxic environments, as well as in other applications where the subject is exposed to high heat stress situations such as fire-fighters, clean room operatives or hospital theatre operatives. In a preferred embodiment, a gas distribution cooling garment system most conveniently comprises a vest which delivers cooling air only to the torso, but may also be a jacket with sleeves, a coverall with sleeves and legs, or any other form which delivers cooling air to specific areas of the body. For optimum comfort and cooling efficiency it is desirable that the garment conforms closely to the body shape of the wearer.

It is an object of the present invention that the cooling gas can be ambient air and that the air can be filtered to remove undesirable components from the cooling air. The cooling gas may also be passed through a heat exchanger to lower the temperature of the gas or through a de-humidifier to further increase its cooling capability. Furthermore, it has been determined that the most efficient cooling using air at an ambient temperature of about 35 °C is achieved by having an air flow of about 4 to 8 liters/second (l/s) over the subject and that the flow should be confined to layer no more than about 4mm from the body of the subject.

Another object of the invention is to provide a high degree of cooling to the wearer, in addition to natural cooling experienced by the wearer, for an extended period of time. Preferably, more than 50 watts of additional cooling is provided over the torso for a period of at least about three hours; more preferably greater than about 80 watts of additional cooling, and further preferred greater than about 100 watts of additional cooling is provided over the torso of a wearer for a period of at least about three hours.

Yet a further object of the invention is that by the use of a gas distribution manifold and a plurality of discrete elements within the cavity defined by the substrates comprising the invention, substantially uniform cooling is achieved over the torso of the wearer.

It is a further object of the invention to provide a personal cooling system that is "non-tethered" and is light- weight. In a preferred embodiment the total weight of the system is less than 3 kilograms.

A further object of the invention is to provide a cooling garment which comprises substrates having high water-vapour-permeability thereby minimising the build-up of perspiration on the wearer's body even when the garment is not supplied with cooling gas.

One embodiment comprising the gas distribution garment of the present invention comprises a first and a second substrate sealed to define at least one cavity. The first substrate is substantially gas-impermeable but water-vapour-permeable. The second substrate is gas-permeable and preferably water-vapour-permeable. The surface of one or both substrates which is orientated towards the inside of the cavity are provided with a plurality of raised protrusions in the form of discrete elements, and the cavity is adapted to contain a gas distribution manifold which is in fluid connection with a gas supply system. The surface of the second substrate external to the cavity is also provided with a plurality of raised protrusions in the form of discrete elements.

In one preferred embodiment, the garment is in the form of a vest, and in use the second substrate will form the inside of the vest such that gas exiting the cavity through the gas-permeable second substrate will flow over the torso of the wearer. The plurality of discrete elements on the surface of the second substrate external to the cavity provides a space between the substrate and either the body of the wearer or any other garment worn thereon. The height of the discrete elements are chosen such that the space between the wearer's body, or any other clothing worn next to the wearer's body, and the gas permeable second substrate is sufficiently wide to allow uniform flow of cooling gas but not so wide that it reduces the cooling effect of the gas. The in-plane spacing between the discrete elements is optimised to distribute the flow of gas exiting the cavity and give substantially uniform cooling of the torso.

The plurality of discrete elements on one or both surfaces of the substrates within the cavity provides a space between the surfaces thereby allowing optimal distribution of the cooling gas within the cavity, and therefore across the wearer's body.

In another embodiment, a gas distribution garment system comprises protrusions external to the cavity that are disposed on an additional substrate that is interposed between the body of the wearer and the external surface of the second substrate forming the cavity. The interposing substrate is preferably water-vapour-permeable and may be gas-permeable. The interposing layer may be attached to the substrates forming the cavity or detached from the cavity substrates.

The plurality of discrete elements contributes to increased conformability of the garment of the present invention by allowing flexing between protrusions compared with prior art garments which utilise mesh or mesh-like spacers. The flexibility of substrates suitable for use in the present invention, having a pattern or plurality of discrete elements thereon, is not substantially less than the flexibility of substrates without any discrete elements. In contrast, the three dimensional structures of the mesh or mesh-like spacers of the prior art lack flex points and they are generally bulky and stiff; therefore the use of these structures results in garments having poor flexibility and conformability.

Furthermore, the plurality of discrete elements also result in a garment construction having lower resistance to gas flow compared with garments of the prior art that utilise mesh or mesh like materials as spacers. Mesh spacers are constructed with material that can interfere with the air flow, whereas materials of the present invention have no intervening material between the discrete elements to interfere with air flow. The low resistance to gas flow afforded by the discrete elements facilitates the use of low power fans to supply cooling gas to the invention and obviates the need for the garment to be "tethered" to a power supply or a high pressure supply of cooling gas. Thus, a preferred embodiment comprises a "portable" or "non-tethered" gas distribution garment system which, as used herein, refers to a system which is not tethered to a (stationary) power supply or a high pressure gas supply. The cooling gas may be ambient air blown into the cavity by battery powered fans which may be optionally fitted with filter elements or other gas treatment systems to remove noxious or other undesirable contaminating components.

### DESCRIPTION OF THE DRAWINGS

Figure **1** represents an embodiment of the invention in the form of a vest and comprising a fan as a means to drive ambient air through a manifold into the cavity of the garment.
Figure **2** is plan view of the body side of the vest illustrating the relative disposition of the discrete elements on the substrate and perforations in the said substrate.
Figure **3** is an enlarged view of area **"X"** in Figure 2 in which the discrete elements comprise round protrusions.
Figure **4** is a representation of the cross-section of an embodiment of the invention wherein the discrete elements within the cavity are disposed on the gas -impermeable substrate.
Figure **5** is a representation of a cross-section of an embodiment of the invention in the direction **Y-Y'** of Figure **2** wherein the discrete elements are disposed either side of the gas- permeable substrate.
Figure **6** is a representation of a gas distribution manifold for use in an embodiment of the invention.
Figure **7** is a representation of an alternative construction of a gas distribution manifold for use in an embodiment of the invention.
Figure **8** shows graphical plots of heart rate (beats/minute) versus time (hours) for a human subject in evaluation trials of an embodiment of the invention.
Figure **9** shows graphical plots of body core temperature for a human subject in evaluation trials of an embodiment of the invention.

### DETAILED DESCRIPTION OF THE INVENTION

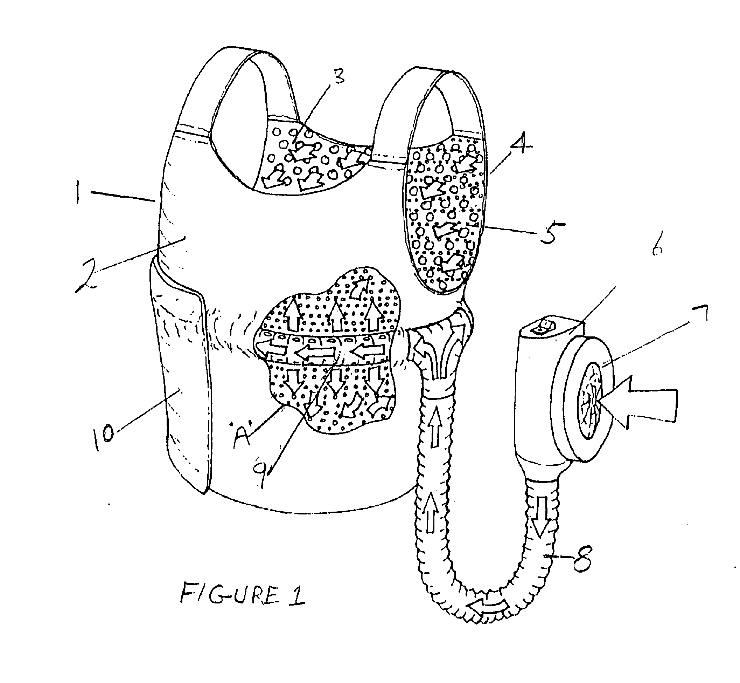

Referring to Figure **1** which represents a preferred embodiment of the present invention the gas distribution cooling garment **1** comprises a substantially gas impermeable substrate **2** attached around it's periphery to a gas permeable substrate **3** to define a cavity, part of which is representationally shown by the cutaway section **A.** Substrate **3** has on its surface, which is external to the cavity and which is proximate to the body of the wearer, a plurality raised protrusions **4** in the form of discrete elements. Substrate **3** is rendered gas-permeable by perforating the substrate between said raised protrusions to give a plurality of holes **5** through which gas can vent from the cavity and pass over the body of the wearer. In one embodiment, the cooling gas is ambient air which is drawn by the fan **6** through optional filter **7** and fed through duct **8** to the air distribution manifold **9** and thence substantially uniformly throughout the volume of the cavity to exit via the perforations **5.** The cooling garment is held in close contact to the body of the wearer by a fastening section **10** which may be fastened using "hook and loop" systems or other suitable methods known in the art.

The direction of air-flow through the system is generally represented by the sequence of block arrows which are included to aid comprehension of the invention and are not to be interpreted as restricting the scope of the invention.

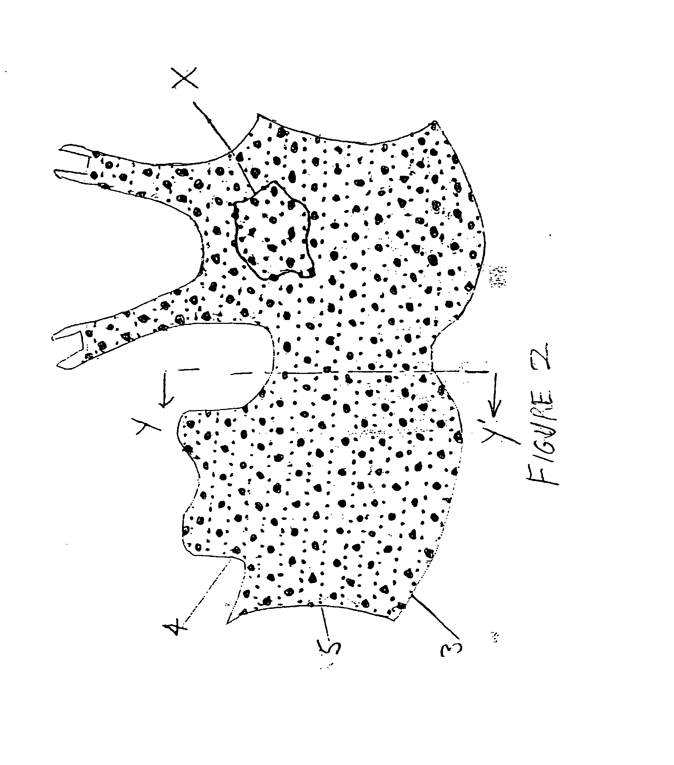

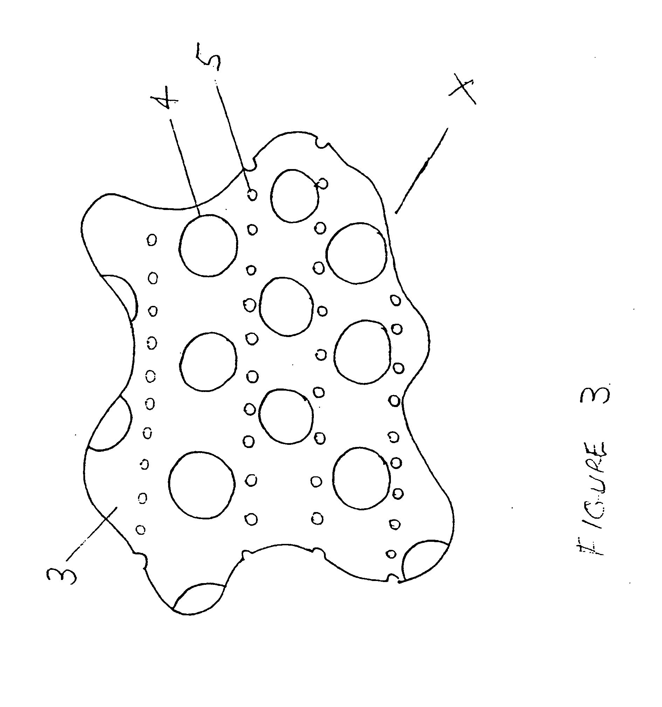

Figure **2** is a plan view of one embodiment of the present invention depicting the surface of the gas permeable substrate **3** that is worn proximate to the body of the wearer. The distribution of the discrete elements **4** and the perforations **5** are more clearly represented and are shown in detail in Figure **3** which is a pictorial enlargement of area **"X"** in Figure **2****.** Illustrative of one embodiment of the present invention, Figure **3** shows the relative distribution (not to scale) of the discrete elements **4** and the perforations **5** on the surface of the substrate **3.** In the embodiment represented, the discrete elements are shown as having circular cross-section in plan view which is not to be seen as limiting the invention. The raised protrusions may comprise other shapes such as cuboidal, conical, pyramidal, polyhedral, hemispherical or truncated hemispherical. By "discrete elements" it is meant a plurality of individual elements, that are substantially or essentially discontinuous or not connected. The discrete elements **4** are preferably soft and resilient but with limited compressibility for optimum comfort and maintenance of air flow. The discrete elements may comprise any material capable of maintaining space between substrate layers, or between a substrate and the body of a wearer, but preferably comprise a thermoplastic or thermosetting polymer selected from, for example, but not limited to silicone, polyester, polyurethane, polyalkene, polyamide, fluoropolymers or other similar materials known to one skilled in the art. Raised protrusions **4** may be applied to substrate **2** by any convenient means such as extrusion or screen printing or other methods known, for example, to one skilled in the art of surface coatings.

For optimal gas flow and cooling the raised protrusions preferably cover 50% or less of the area of the surface of substrate **3** which is proximate to the body of the wearer, a preferred coverage is less than 30% of the surface area and a more preferred coverage is less than 20%. It has been discovered by the inventor that, surprisingly, optimal cooling is achieved in systems wherein the height of the raised protrusions, preferably in the form of discrete elements, **4** is in the range of about 1mm to 20mm, preferably in the range about 2mm to 10 mm and more preferably in the range about 2mm to 4mm. Preferably, the raised protrusions **4** define a plurality of channels having a depth equivalent to the height of the protrusions, between the external surface of substrate **3** and the wearer. The cooling gas which exits through perforations **5** flows through the aforesaid channels and is distributed substantially uniformly over the body of the wearer.

The perforations **5** shown as circular in cross section may also be of other cross-sections and are preferably uniformly distributed over the surface of substrate **3** to ensure uniform gas-flow over the body of the wearer. The cross-sectional area of a single perforation is preferably equivalent to that of a circular perforation having a diameter of between about 1 mm and 2 mm. The perforations should be sufficient in number for substrate **3** to have an air permeability preferably of between about 10 and 100 l m⁻²s⁻¹ at a pressure drop of about 100 Pa and more preferably of between about 60 and 70 l m⁻² s⁻¹ at a pressure drop of about 100 Pa .

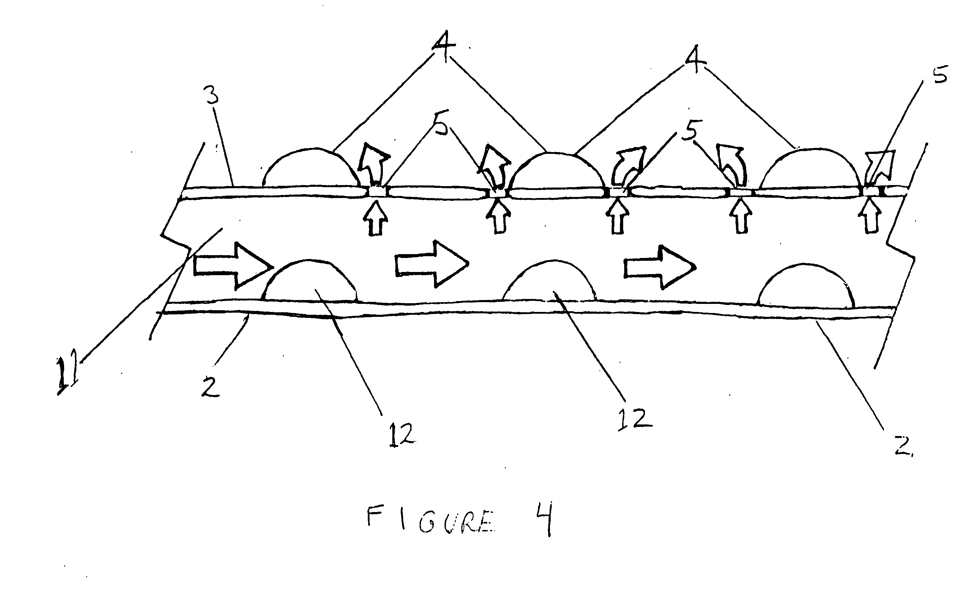

Figure **4** shows enlarged detail of a cross-sectional view in the direction of **Y-Y'** of Figure **2** of an embodiment of the invention. Substrates **2** and **3** define cavity **11** into which the cooling gas is passed from the gas distribution manifold (not shown). The raised protrusions **4** which comprise discrete elements having a hemispherical profile are provided on the external surface of substrate **3,** i.e. the surface which is external to cavity **11.** When the garment is worn the protrusions **4** are in contact with the body of the wearer or in contact with an article of clothing, such as underwear or t-shirt, worn by the wearer.

Referring again to Figure **4** it will be seen that this embodiment comprises a plurality of raised protrusion integral with the surface of substrate **2,** disposed internal to cavity **11.** These are in the form of hemispherical discrete elements **12** which are uniformly distributed over the surface of substrate **2** within cavity **11.** Raised protrusions preferably in the form of discrete elements **12** cover preferably less than 50% of the area and, more preferably, less than 30% of the surface of substrate **2** which is internal to the cavity. A function of the discrete elements **12** disposed within the cavity, is to act as spacer members to prevent the collapse of cavity **11,** for example, when heavy articles of clothing or a self contained breathing apparatus is worn over the cooling garment of the invention. A further function of the discrete elements **12** is to aid in the uniform distribution of the cooling gas throughout the cavity **11.**

The height of the discrete elements **12** within the cavity is preferably in the range of about 1mm to 20mm. To minimise the thickness of the vest, and maximise its conformability and flexibility, and to ensure uniform distribution of the cooling gas through the cavity **11,** a preferred height of the discrete elements may range from about 2mm to 10mm. The discrete elements **12** located within the cavity may comprise any suitable material but preferred materials are soft, resilient polymers having limited compressibility. The polymers may be thermosetting or thermoplastic and may be selected from a range of polymers such as silicones, polyurethanes, polyesters, polyamides , polyalkenes fluoropolymers or other polymers deemed suitable by one skilled in the art, and may be applied to the supporting substrate by extrusion, screen printing or any suitable method known to one skilled in the art.

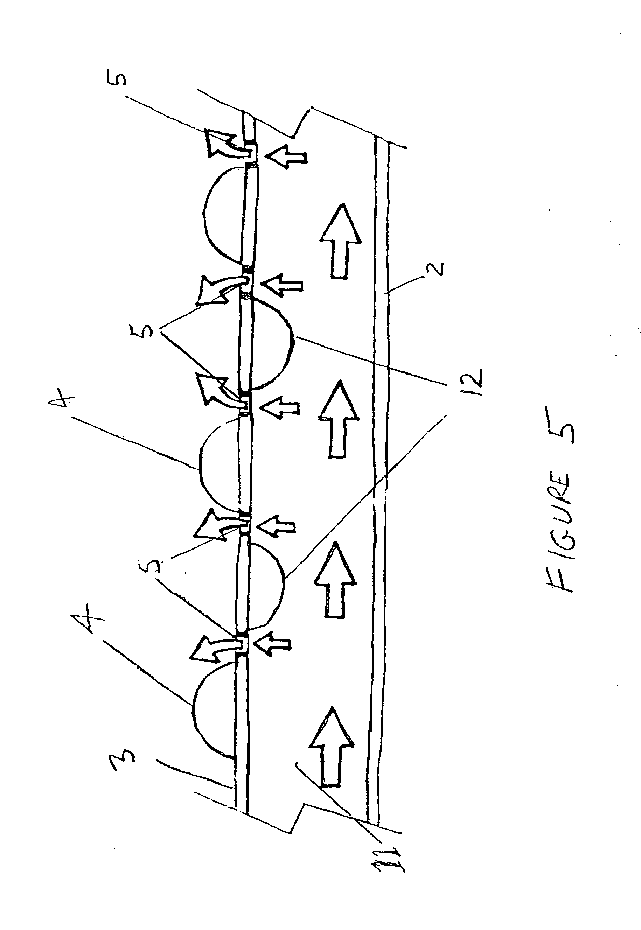

A further embodiment of the invention is shown in Figure **5** which is a cross-section of a garment having an alternative arrangement of raised protrusions in the form of discrete elements **12** within the cavity **11.** In this embodiment the discrete elements **12** are located on the internal surface of substrate **3** and are positioned so as to be off-set from the protrusions **4** which are situated on the opposite surface of substrate **3.** In a further embodiment the discrete elements **12** on the internal surface of substrate **3** may be in alignment with the position of protrusions **4** on the external surface of the substrate, while maintaining airflow through the perforations **5.**

Substrate **2** is preferably substantially gas impermeable; by "substantially gas impermeable" is meant a substrate having less than about 10% of the gas permeability of the gas permeable second substrate. Preferred substrates have an air permeability of less that 10 lm⁻²s⁻¹ at pressure of 100 Pa. Preferably, substrate **2** is also water vapour permeable. Substrate **3** may be a gas impermeable layer which has been perforated, or may be an intrinsically air permeable layer such as a laminate of microporous PTFE, a tightly woven textile, or a dense non-woven textile, with preferred constructions comprising an air permeability in the range of between about 10 and 100 l m⁻²s⁻¹ as previously taught herein. Where perforated, substrate **3** may be rendered somewhat water-vapour-permeable by the perforations **5** but it is preferred that the material of construction of substrate **3** is inherently water-vapour permeable.

Substrates **2** and **3** may comprise single monolithic constructions or may comprise a plurality of layers of different materials chosen to impart the desired features to the substrates, such as air permeability and water vapour permeability. A preferred construction is a laminate of knitted or woven textile and an expanded polytetrafluoroethylene membrane coated with a water vapour permeable polymer. Such laminates are sold under the GORE-TEX^{®} trade name by W.L. Gore and Associates Inc. Newark DE. Preferred water vapour permeable materials for use in the substrates of the present invention including both the gas impermeable substrate and the gas permeable substrate, may be comprised of a layer of a water-vapour permeable polymer such as polyurethane, polyester or microporous polyurethane or may comprise such polymers coated on or laminated to a textile construction. Preferred materials are those having water vapor evaporative resistance (Ret) values less than about 20 m² Pa W⁻¹ as measured according to ISO 11092. More preferred materials are those having Ret values less than about 15m² Pa W⁻¹ as measured according to ISO 11092.

For maximum flexibility and conformability to the wearer's body-shape the substrates 2 and 3 should be as thin as possible whilst having sufficient robustness to withstand the stresses of use. Substrate 3 may comprise a monolithic single layer construction or a plurality of layers or a laminate comprising the same or different material that is chosen for substrate 2.

In an alternate construction of the present invention, a gas distribution garment system is formed wherein the plurality of raised protrusions external to the cavity are not disposed directly on the external cavity surface. The raised protrusions external to the cavity surface are disposed on an additional substrate that is interposed between the body of the wearer and the external surface of the cavity. In a first embodiment of this alternate construction, the plurality of raised protrusions external to the cavity surface are disposed on an additional substrate located between the external cavity substrate and the skin of the wearer, and the raised protrusions are predominantly oriented towards the skin. The additional substrate may be any suitable woven, non-woven or knitted fabric which is air permeable. For example, a knitted undergarment worn separately from the gas distribution garment may comprise a plurality of raised protrusions disposed on the inside of the undergarment directed toward the skin of the wearer. In this preferred embodiment, the additional substrate comprising the raised protrusions is air permeable to enable the flow of air from the air permeable cavity substrate to flow through the additional substrate into close proximity with the wearer's skin.

In a second embodiment of this alternate construction, the additional substrate comprising the plurality of raised external to the cavity is also located between the body of the wearer and the external surface of the cavity. In this embodiment the plurality of raised protrusions are predominantly disposed on the additional substrate in an orientation that is away from the skin. The additional substrate may be any suitable woven, non-woven or knitted fabric which is water vapor permeable such as, for example, a knitted undergarment such as a T-shirt. In this embodiment, the additional substrate is water vapor permeable to permit the evaporation of water from the skin into the stream of air which is formed external to the cavity between the air permeable cavity substrate and the additional substrate comprising the raised protrusions. In this embodiment, the additional substrate is optionally air permeable. In an embodiment of the alternate construction of the present invention, the additional substrate may be permanently affixed to one or both of the substrates that form the cavity, or the additional substrate may be detachably affixed to the substrates, or the additional substrate may be separate from the substrates that form the cavity.

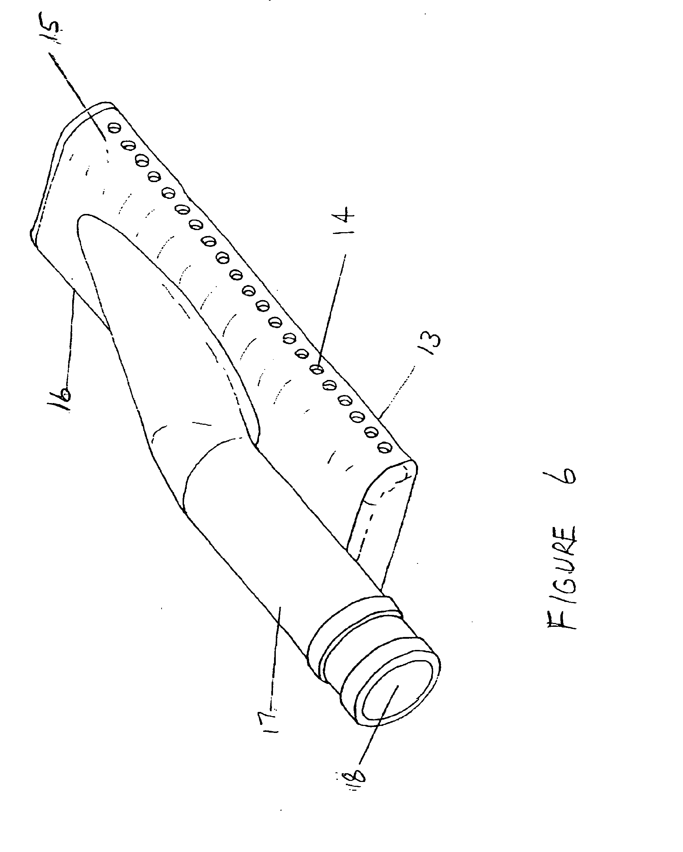

The cavity formed by the substrates is adapted for connection with a gas supply so that gas flows into the cavity and exits the cavity through the gas permeable substrate. A preferred means for such adaptation comprises a gas distribution manifold, substantially hollow in cross section, which is in fluid connection with the gas supply and comprises a series of perforations to allow gas to be distributed within at least part of the cavity. Figure **6** is a representation of the construction of a gas distribution manifold for use in an embodiment of the invention. The manifold of Figure **6** comprises a hollow elongate member **13** which is substantially rectangular in cross-section, though it should be understood that other cross-sectional shapes are suitable for use in the present invention Hollow member **13** is provided with a series of perforations **14** along the sides **15** and **16** and a gas feed duct **17.** In use hollow member **13** is placed in the cavity of the garment with the duct **17** external to the cavity. The cooling gas is fed through the lumen **18** of duct **17** and is distributed into the cavity of the garment through perforations **14.**

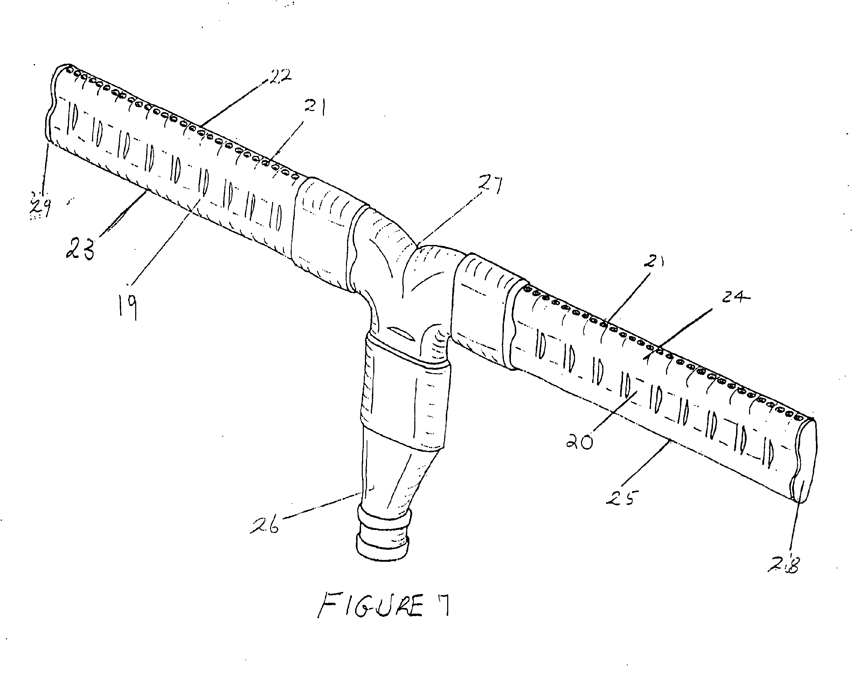

Figure **7** is a representation of an alternative and preferred construction of a gas distribution manifold for use in the garment of the invention and corresponds to item **9** in Figure **1****.** The construction comprises two hollow elongate members **19** and **20** which are substantially cylindrical in cross-section and have a series of perforations **21** along edges **22, 23, 24,**and **25.** Hollow members **19** and **20** are connected to gas feed duct **26** by union piece **27** and the ends members **19** and **20** remote from union piece **27** are closed off by blanking pieces **28** and **29.**

In use, members **19** and **20** are preferably placed in the cavity of the garment such that one member is in the area of the garment which covers the front of the torso of the wearer and the other member is in the back area of the garment. Gas feed duct **26** is the representative embodiment is external to the cavity of the garment. Cooling gas fed into duct **26** is fed into both members **19** and **20** and is distributed into the cavity through perforations **21.**

The members **19** and **20** maybe constructed of any suitable material known to one skilled in the art but for optimal comfort for the wearer the material should be soft and flexible and preferably resilient with only a slight degree of compressibility. Suitable materials include elastomeric materials such as polyurethane, polyester, or synthetic rubbers such as EPDM or SBR. It is preferable for the material to have a hardness in the range of 55-65 Shore A.

### EXAMPLES

### EXAMPLE 1

To demonstrate the efficacy of an embodiment of the invention a garment was constructed according to the teaching of this specification and its cooling effectiveness evaluated whilst being worn by a human subject walking on a tread-mill.

The first and second substrates comprised a laminate of Basofil^{®} spun bonded non-woven textile and expanded polytetrafluoroethylene having an air-impermeable water vapour permeable coating with a plurality of foamed silicone rubber protrusions uniformly distributed on the Basofil^{®} surface. The laminate is available from W.L. Gore and Associates GmbH, Putzbrunn, Germany under the trade name Airlock^{®} Part No. AIRL 002000. The silicone rubber protrusions are approximately 3mm in height and cover an area of approximately 13% of the surface of the laminate.

Two pieces of Airlock® AIRL 002000 laminate were cut and sized according to Figure **2** to give a body coverage of about 0.45m². The laminate corresponding to the second substrate of the invention was perforated with a 1.34 mm diameter needle to give a grid pattern of holes on an approximately 6mm by 10mm spacing. The air permeability of the laminate resulting from the perforations was about 60 l.m⁻²s⁻¹ at a pressure drop of about 100 Pa.

The cut pieces of laminate were oriented according to the arrangement in Figure **4** and attached round their periphery by sewing, thereby forming a cavity. A gas distribution manifold of the general arrangement of Figure 7 was formed from two lengths of 25 mm inside diameter cylindrical cable duct (Part No. 364-3458 from RS Components Ltd. Corby, Northants, England) corresponding to members **19** and **20** of Figure **7****.** The length of each member was about 460mm. A uniform series of approximately 4mm diameter holes were drilled in the surfaces of the duct corresponding to surfaces **22, 23, 24** and **25** of Figure **7** to give 92 holes per member. The ends of the duct within the cavity were sealed with blanking pieces and the other ends terminated in a union piece and gas entry duct corresponding to **27**and **26** respectively of Figure **7****.** An electrically powered fan, (Part No. U97EM-012KK-3 from Acal Radiatron, Egham, Surrey, England) was connected to the gas duct to complete the assembly. During the evaluation trials, for convenience, the fan was powered from a bench mounted power supply unit adjusted to provide about 15 Volts dc to the fan. With this set up the airflow from the fan was calculated by measuring the pressure drop across the fan and comparing this with the pressure drop versus flow from the manufacturers data sheet for the fan. The flow was ascertained to be about 10 litres/sec.

For the evaluation trials the subject was clad in the following manner. The subject was dressed in a cotton T-shirt and cotton briefs next to the skin. The cooling garment of Example 1 was provided over the T-shirt. Over the cooling garment, a British Army Mk IV protective suit was provided. Finally, on top of the protective suit, a British Army MK I Fragmentation vest was provided. The feet were covered in socks and heavy boots, and the hands were covered with lightweight cotton gloves under rubber gloves. A respiration mask was placed on the face of the subject.

Three evaluation trials were performed in the following manner.

Trial 1 - Subject clad as above with fan running (i.e. cooling in operation).

Trial 2 - Subject clad as above with fan switched off (i.e. no cooling).

Trial 3 - Subject clad as above but cooling garment removed (i.e. garment ensemble as currently used by military personnel remained).

The subject was tasked to walk on a tread-mill set at a linear speed of about 4.5 km/hr and the subject's body core temperature and heart rate monitored and recorded. The duration of each trial consisted of periods of about 100 minutes of walking followed by rest periods of about 30 minutes. The evaluation trials were carried out in an environmentally conditioned room at an ambient temperature of approximately 35 °C and a relative humidity of 50%.

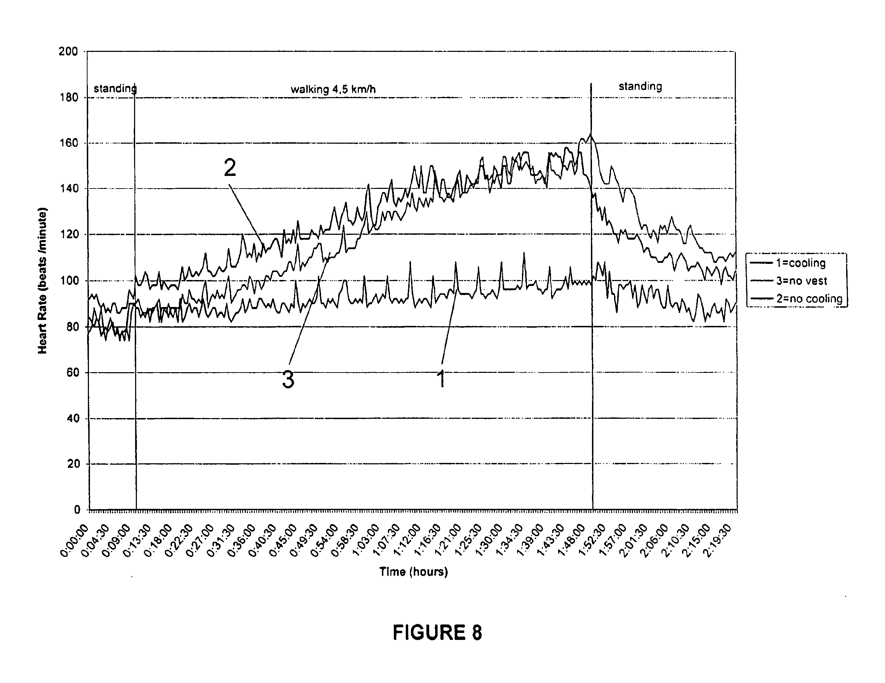

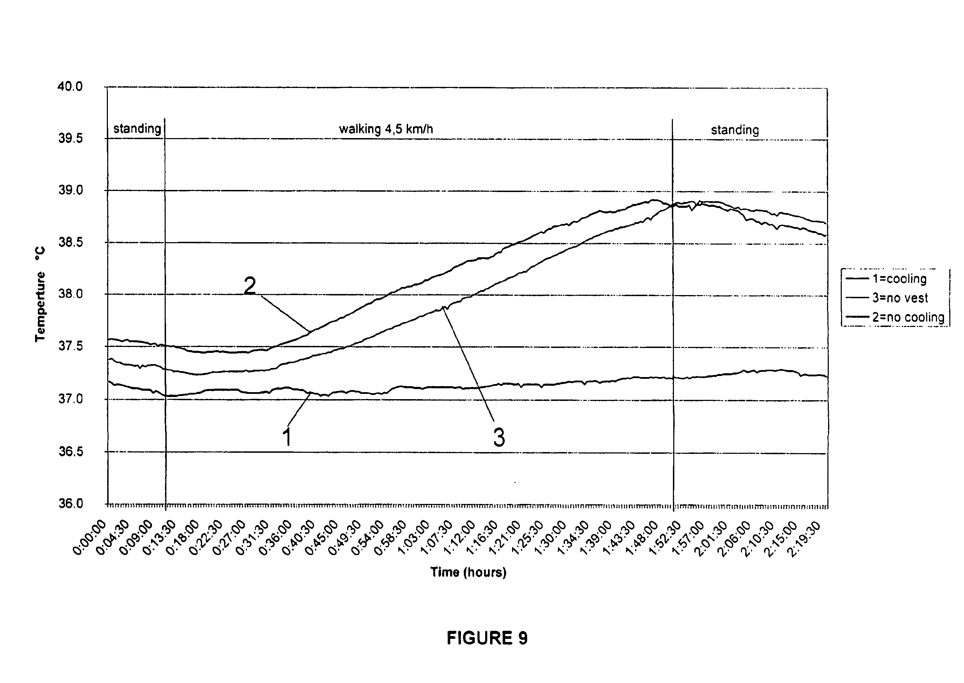

The plot of heart rate versus time and the plot of body core temperature versus time for all three trials are shown respectively in Figures **8** and **9****.** Referring to Figure **8****,** the plot of heart rate (beats/minute) versus time (hours), shows the highly significant cooling effect of the garment of the invention. The plot of heart rate against time for the subject with the garment in cooling mode ("cooling" plot) corresponding to Trial 1 shows a slight overall rise in heart rate (from approximately 80 beats per minute to approximately 100 beats per minute) throughout the duration of the trial. The regular peaks in the plot correspond to the exercise periods but, with the cooling in operation, the rate drops back to substantially the base level during the rest periods. In contrast, however, the heart rate plots for the "no cooling" and the "no vest" modes (Trials 2 and 3) result in regular rise in heart rate throughout the trials from approximately 80 beats per minute to highly undesirable rates of 160 beats per minute.

The close correlation between the plots for Trials 2 and 3 does however demonstrate another highly desirable feature of the invention i.e. that even when the garment is worn without it being cooled it adds little or nothing to the thermo-physiological load on the wearer compared with the clothing ensemble not including the cooling garment.

The body core temperature plots in Figure **9** further confirms the effectiveness of the cooling garment of the invention. The "cooling" plot shows the very small rise (less than about 0.5 °C) in the subject's body core temperature. In contrast, the "no cooling" and "no vest" plots corresponding to Trials 2 and 3 show highly undesirable increases of almost 2 °C. However, as with the heart rate plots, the body core temperature plots demonstrate the negligible thermo-physiological loading characteristics of the garment when worn without the cooling in operation.

The objectives of the invention are also clearly achieved by the garment of the above example. Whereas in the foregoing trials the fan was powered by a bench mounted power supply unit it has been shown that a battery powered fan could be used and the same air flow rates achieved. The fan of the example was replaced by a fan requiring only a 5 Volt dc supply (Part no.U97LM-005K1 from Acal Radiatron, Egham, Surrey, England) and the replacement fan powered by a nominally rated 6.4 Volt battery with an underload voltage of 5.0 Volts (Part no. U3356H/2/7, from Ultralife Batteries Ltd. Abingdon, Oxfordshire, England.) The fan gave an output of about 6 litres/sec for over 9 hours.

The garment of the example with the fan and battery attached weighed approximately 2.1kg, which is considerably less than the 3kg target for a lightweight system.

### EXAMPLE 2

### Cooling

To evaluate the cooling power of the cooling garment prepared substantially according to Example 1, it was subject to Thermally Instrumented Manikin testing by The Cord Group Ltd., Dartmouth, Nova Scotia, Canada. The cooling garment was tested in combination with a standard British Army Mk IV protective suit as used in the foregoing Example 1 and under the various conditions as detailed in the following Table 1. Testing was carried out in a temperature and humidity controlled room with an ambient temperature set at 35 °C and relative humidity set 50%. Details of the test methodology are as follows.

### Test Method

The evaluation of cooling vest prototypes using UK standard suit ensemble was conducted using a Thermal Instrumented Manikin Test System. During the testing, environment temperature, skin temperature and power consumption were recorded.

The Thermal Manikin Test System consists of a hollow aluminum manikin equipped with temperature sensors and electric heaters connected to a computer system. The manikin was dressed in the human-use apparel to be tested and placed in an appropriate environment. The computing equipment controlled the heaters to maintain the skin of the manikin at a set temperature and measured the electrical power required to do so. This power is equivalent to the heat that escaped through the clothing due to the temperature difference across it. The power and the temperature difference were then used, along with the known surface area of the manikin to calculate the thermal resistance offered by the apparel.

The thermal performance of a garment was evaluated by unmanned tests on the whole garment under conditions identical or similar to actual operating conditions. The system employed a life-sized watertight manikin capable of being heated to and maintained at a.selected temperature.

The system comprised a Thermally Instrumented Manikin (TIM), a control module, a computer, environmental temperature sensors and cables connecting these components. The manikin was in a shape of human proportions to fit inside the test garment. The combinations of the aluminum shell of the manikin and the output of heaters inside it provided for an approximately uniform temperature over the manikin surface. This temperature is sensed by sensors embedded in the manikin's shell and is then passed to the control module.

The control module housed the programmed data acquisition system, the heater relays and other circuit components. The data acquisition system received data from the temperature sensors on the manikin and controlled the heater relays so that the manikin surface temperature remains constant. It also measured the environment temperature and the power applied to the manikin and was programmed with the surface area of the manikin. With this temperature, power and area data, it calculated the insulation value of the garment and passed this, along with other pertinent data to the computer. The computer acted as a control and display terminal and post-processor.

The following clothing combination was used for testing. The manikin was first covered in a shirt with long sleeves and trousers assembled into a coverall (skin) made of an interlock knit (high stretch), white 100% cotton textile. Tubes for the distribution of water were sewn into the garment. Depending on the test set-up as described in Manikin Set Up, A through E, of Table 1 cooling garments prepared according to Example 1 (two styles) were selected and optionally provided over the coverall. One style of cooling garment comprised a single entry port manifold (Fig. 6), and a second style of cooling garment was provided with a split manifold (Fig. 7). An outer layer comprising a UK Standard protective suit ensemble top and bottom, and a Mk I ballistic vest, was provided over the cooling garment, or depending on test conditions, directly over the coverall (skin). Garment openings were secured as follows. Arm cuffs were tucked and secured with elastic straps; front zippers were secured to the top; and bottom of legs were secured with elastic straps. Tensioning straps on the ballistic vest were secured.

The manikin was lifted into a vertical position and suspended in the test chamber hanging from a head bolt with feet lightly touching the floor. Environmental sensors were suspended around the manikin to detect the environment temperature. The manikin temperature was set at about 35.0 °C. The ambient temperature of the chamber was set at about 35 °C and actual temperature was measured at about 34.16 - 34.31 °C. The ambient relative humidity of the chamber was set at about 50% about and measured at about 48.5 - 56.0 %. Water, fed to the cotton garment by way of the tubes, was provided to simulate wetting by sweat. A warm-up period was provided to allow the manikin to reach the set temperature and go into test period. The long-term power was monitored for all calculated sections until steady state condition was reached, and the test was restarted.

The steady state long term power results of the thermal instrumented manikin with and without gas distribution vests of the present invention and standard British protective suit ensemble is as follows.

**Table 1.**

| Manikin Set Up | Description | Air Flow l/s | Long Term Power (watts) | | | | |
|---|---|---|---|---|---|---|---|
| | | | Front* | Back** | Arms | Legs | Overall |
| A | Protective suit with ballistic vest, skin wet, no cooling vest, no cooling baseline | 0 | 3.42 | 1.10 | 23.88 | 41.30 | 69.71 |
| B | Protective suit with ballistic vest, skin wet, split duct cooling vest, no cooling | 0 | 2.70 | 1.17 | 21.40 | 42.92 | 68.19 |
| C | Protective suit with ballistic vest, skin wet, split duct cooling vest,cooling @ 15 v dc | 9.28 | 41.45 | 49.74 | 24.78 | 102.39 | 218.36 |
| D | Protective suit with ballistic vest, skin wet, CZ15 single entry cooling vest, cooling @ 15 v | 9.36 | 46.17 | 49.51 | 32.46 | 93.68 | 221.82 |
| E | Same as test number D with backpack added with 102 lbs contained in pack | 8.71 | 44.79 | 47.45 | 26.67 | 94.59 | 213.50 |

| | | | | | | | |
|---|---|---|---|---|---|---|---|
| Front * - Consists of chest and abdomen sections of the manikin Back ** - Consists of back and buttocks sections of the manikin | | | | | | | |

Table 1 illustrates the significant overall cooling power of the cooling garment of the present invention when energised in cooling mode. Furthermore, a comparison of the results of Manikin Set Ups A and B demonstrates the minimal additional thermal stress added to the Thermally Instrumented Manikin by the cooling garment system of the invention when the garment is not energised for cooling.

### Conformability

Conformability of the garment of the present invention was tested and compared with a mesh spacer material representative of those used by garments of the prior art. A sample comprising Airlock® Laminate AIRL 02000 was prepared according to the air permeable second substrate of Example 1 having a plurality of protrusions and perforations, and was tested and compared with spacer material from Mueller Textile Germany, Mueller Part no. 5911.

### Test Method

The test method used was performed substantially as described in ASTM D 4032 - 94 (as re-approved in 2001) - Standard Test Method for Stiffness of Fabric by the Circular Bend Procedure, with the following modifications. The size of the test sample was 4 inches by 4 inches (100mm by 100mm) an Instron Model 1011 tensile/compression tester operating with Instron Series 9 software replaced the force measurement gauge; and the plunger speed was set at 500mm/min.

The Airlock® laminate was tested in three different modes, as follows:
Trial 1: laminate was tested on its own with the protrusions facing downwards in contact with the test platform;
Trial 2: laminate was tested on its own with the protrusions facing upwards in contact with the plunger;
Trial 3: laminate was tested in combination with an 84g/m² woven polyester face fabric to simulate a garment construction of the invention .

Five samples of each material were subjected to the conformability test and results are summarised below, in Table 2.

**Table 2.**

| Material: (Trial#) | AIRL 02000 (Trial 1) | AIRL 0200 (Trial 2) | AIRL 02000 (Trial 3) | Spacer material (Mueller 5911) |
|---|---|---|---|---|
| Average peak force (kg) | 0.010 | 0.011 | 0.009 | 0.049 |

The differences between the conformability of the materials of the present invention compared with other spacer material are clearly demonstrated by this test. Materials having lower average peak force values are deemed more conformable than materials having higher average peak force values. Thus, preferred embodiments of the present invention comprise a conformability peak force value of preferably less than or equal to 0.03 kg, more preferably less than or equal to 0.02 kg, and further preferred, less than or equal to 0.01 kg, for a substrate comprising a plurality of raised protrusions on a substrate surface, when tested according to the method provided herein.

Whereas the foregoing examples are demonstrative of a specific embodiment of the invention it should not be deemed to be limiting in scope. One skilled in the art will select other embodiments, designed for specific end uses. For example an embodiment of the invention intended for use by fire fighters and other operatives subjected to fire or other high temperature situations may comprise non-melting and non-flammable materials.

While particular embodiments of the present invention have been illustrated and described herein, the present invention should not be limited to such illustrations and descriptions. It should be apparent that changes and modifications may be incorporated and embodied as part of the present invention within the scope of the following claims.

## Claims

1. A garment (1) for cooling the body of a wearer comprising:
a substantially gas impermeable first substrate (2) and a gas-permeable second substrate (3) attached around substrate peripheries forming a cavity (11) therebetween, wherein the cavity (11) is adapted to be connected to a gas supply such that the gas flows into the cavity (11) and exits the cavity (11) through the gas permeable second substrate (3); **characterized in that**
at least one of the first and second substrates (2, 3) comprising a plurality of raised protrusions (12) on a surface within the cavity (11), and
the gas permeable second substrate (3) comprising a plurality of raised protrusions (4) on the surface external to the cavity (11) and proximate to the body of the wearer;

2. The garment of claim 1 wherein the first substrate comprises a laminate.

3. The garment of claim 1 wherein the second substrate comprises a laminate.

4. The garment of claim 1 wherein the first and second substrates both comprise a laminate.

5. The garment of claim 1 wherein the first and second substrates are water-vapour-permeable.

6. The garment of claim 2 wherein the first and second substrates are water-vapour-permeable laminate.

7. The garment of claim 1 wherein the second substrate comprises perforations.

8. The garment of claim 1 wherein the second substrate comprises a microporous membrane.

9. The garment of claim 1 wherein the second substrate comprises a woven textile.

10. The garment of claim 1 wherein the second substrate comprises a non-woven textile.

11. The garment of claim 1 wherein the first and second substrates have an evaporative resistance value of less than 20 m² Pa W⁻¹.

12. The garment of claim 1 wherein the first and second substrates have an evaporative resistance value of less than 15m² Pa W⁻¹.

13. The garment of claim 1 wherein the second substrate has an air-permeability of between 10 and 100 litres/m² /sec. at a pressure of 100Pa.

14. The garment of claim 1 wherein the gas flows over the wearer's body at a rate of between 1 and 10 litres/second.

15. The garment of claim 1 wherein the gas flows over the wearer's body at a rate of between 4 and 8 litres/second.

16. The garment of claim 1 wherein the raised protrusions are in the form of discrete elements.

17. The garment of claim 1 wherein the raised protrusions comprise a thermoplastic polymer.

18. The garment of claim 1 wherein the raised protrusions comprise a thermosetting polymer.

19. The garment of claim 1 wherein the raised protrusions on the second substrate have a height of between 1 mm and 20mm.

20. The garment of claim 1 wherein the raised protrusions on the second substrate have a height of between 2mm and 4mm.

21. The garment of claim 16 wherein the spacing between the discrete elements is between 5mm and 25mm.

22. The garment of claim 20 wherein the raised protrusions are discrete elements and the spacing between the discrete elements is between 5mm and 25mm.

23. The garment of claim 1 wherein the raised protrusions within the cavity are located on the first substrate.

24. The garment of claim 1 wherein the raised protrusions within the cavity are located on the second substrate.

25. The garment of claim 1 wherein the first substrate comprises PTFE.

26. The garment of claim 1 wherein the second substrate comprises PTFE.

27. The garment of claim 25 wherein the PTFE comprises expanded PTFE.

28. The garment of claim 26 wherein the PTFE comprises expanded PTFE.

29. The garment of claim 1 wherein the first and second substrates are flexible.

30. The garment of claim 1 wherein the gas permeable second substrate is conformable.

31. The garment of claim 1 wherein a substrate comprising a plurality of raised protrusion on a surface has a conformability peak force of less than or equal to 0.03kg.

32. The garment of claim 1 wherein the gas permeable second substrate comprising a plurality of raised protrusion on a surface has a conformability peak force of less than or equal to 0.03kg.

33. The garment of claim 1 wherein the gas permeable second substrate comprising a plurality of raised protrusion on a surface has a conformability peak force of less than or equal to 0.02kg.

34. The garment of claim 1 wherein the gas permeable second substrate comprising a plurality of raised protrusion on a surface has a conformability peak force of less than or equal to 0.01 kg.

35. The garment of claim 1 wherein the gas flow into said cavity comprises ambient air.

36. The garment of claim 1 wherein the garment is non-tethered.

37. The garment of claim 1 wherein the gas flowing into the cavity is filtered or otherwise treated to remove undesirable components.

38. The garment of claim 1 wherein said cavity comprises a gas distribution manifold.

39. The garment of claim 1 which comprises non-flammable materials.

40. The garment of claim 1 which comprises non-melting materials.

41. A system for cooling the body of a wearer comprising
a garment (1) comprising a substantially gas impermeable first substrate (2) and a gas-permeable second substrate (3) attached around substrate peripheries forming a cavity (11) therebetween, wherein the cavity (11) is adapted to be connected to a gas supply such that the gas flows into the cavity (11) and exits the cavity (11) through the gas permeable second substrate (3); **characterized in that**
at least one of the first and second substrates (2, 3) comprising a plurality of raised protrusions (12) on a surface within the cavity (11), and
at least one additional substrate interposed between the body of the wearer and the gas permeable second substrate (3) of the garment (1), the at least one additional substrate comprising a plurality of raised protrusions on a substrate surface;

42. The system according to claim 41 wherein the at least one additional substrate is affixed to the garment.

43. The system according to claim 41 wherein the at least one additional substrate is detachably affixed to the garment.

44. The system according to claim 41 wherein the at least one additional substrate and the garment are separate.

45. The system of claim 41 wherein the first substrate comprises a laminate.

46. The system of claim 41 wherein the second substrate comprises a laminate.

47. The system of claim 41 wherein the at least one additional substrate comprises a laminate.

48. The system of claim 41 wherein the first and second substrates both comprise a laminate.

49. The system of claim 41 wherein the first substrate is water-vapour-permeable.

50. The system of claim 41 wherein the second substrate is water-vapour permeable.

51. The system of claim 41 wherein the second substrate comprises perforations.

52. The system of claim 41 wherein the second substrate comprises a microporous membrane.

53. The system of claim 41 wherein the at least one additional substrate comprises a knit, woven or non-woven textile.

54. The system of claim 41 wherein the at least one additional substrate is water-vapor permeable.

55. The system of claim 54 wherein the at least one additional substrate is a gas permeable.

56. The system of claim 55 wherein the at least one additional substrate comprises perforations.

57. The system of claim 55 wherein the at least one additional substrate comprises raised protrusions oriented towards the body of a wearer.

58. The system of claim 54 wherein the at least one additional substrate comprises raised protrusions oriented away from the body of a wearer.

59. The system of claim 58 wherein the at least one additional substrate is gas permeable.

60. The system of claim 41 wherein the at least one additional substrate comprises an undergarment.

61. The system of claim 41 wherein the at least one additional substrate comprises a T-shirt.

62. The system of claim 41 wherein the at least one additional substrate has an evaporative resistance value of less than 20 m² Pa W⁻¹.

63. The system of claim 41 wherein the at least one additional substrate has an evaporative resistance value of less than 15m² Pa W^{-1.(?)}

64. The system of claim 55 wherein the at least one additional substrate has an air-permeability of between 10 and 100 litres/M² /sec. at a pressure of 100Pa.

65. The system of claim 41 wherein the flow of gas over the wearer's body is between 1 and 10 I/sec.

66. The system of claim 41 wherein the flow of gas over the wearer's body is between 4 and 8 I/sec.

67. The system of claim 41 wherein the raised protrusions are in the form of discrete elements.

68. The system of claim 41 wherein the raised protrusions comprise a polymer.

69. The system of claim 41 wherein the raised protrusions comprise a thermoplastic polymer.

70. The system of claim 41 wherein the raised protrusions comprise a thermosetting polymer.

71. The system of claim 41 wherein the raised protrusions have a height of between 1mm and 20mm.

72. The system of claim 41 wherein the raised protrusions on the at least one additional substrate have a height of between 2mm and 4mm.

73. The system of claim 41 wherein the spacing between the raised protrusions is between 5mm and 25mm.

74. The system of claim 72 wherein the spacing between the raised protrusions is between 5mm and 25mm.

75. The system of claim 41 wherein the first substrate comprises PTFE.

76. The system of claim 41 wherein the second substrate comprises PTFE.

77. The system of claim 75 wherein the PTFE comprises expanded PTFE.

78. The system of claim 76 wherein the PTFE comprises expanded PTFE.

79. The system of claim 41 wherein the first and second substrates are flexible.

80. The system of claim 41 wherein the at least one additional substrate is conformable.

81. The system of claim 41 wherein a substrate comprising a plurality of raised protrusions has a conformability peak force of less than or equal to 0.03kg.

82. The system of claim 41 wherein the gas permeable second substrate comprises a plurality of raised protrusions and has a conformability peak force of less than or equal to 0.03kg.

83. The system of claim 41 wherein the gas permeable second substrate comprises a plurality of raised protrusions and has a conformability peak force of less than or equal to 0.02kg.

84. The system of claim 41 wherein the gas permeable second substrate comprises a plurality of raised protrusions and has a conformability peak force of less than or equal to 0.01 kg.

85. The system of claim 41 wherein the gas flow into said cavity comprises ambient air.

86. The system of claim 41 wherein the gas flowing into the cavity is filtered or otherwise treated to remove undesirable components.

87. The system of claim 41 wherein said cavity comprises a gas distribution manifold.

88. The system of claim 41 which comprises non-flammable materials.

89. The system of claim 41 which comprises non-melting materials.

90. The system of claim 41 wherein the system is non-tethered.

## Patentansprüche

1. Kleidungsstück (1) für die Abkühlung des Körpers eines Trägers, das aufweist:
ein im Wesentlichen gasundurchlässiges erstes Trägermaterial (2) und ein gasdurchlässiges zweites Trägermaterial (3), das um den Umfang der Trägermaterialien herum befestigt ist, wodurch ein Hohlraum (11) dazwischen gebildet wird, wobei der Hohlraum (11) so angepasst ist, dass er mit einer Gaszuführung verbunden wird, so dass das Gas in den Hohlraum (11) strömt und aus dem Hohlraum (11) durch das gasdurchlässige zweite Trägermaterial (3) austritt;
**dadurch gekennzeichnet, dass**
mindestens eines von erstem und zweitem Trägermaterial (2, 3) eine Vielzahl von erhabenen Vorsprüngen (12) auf einer Oberfläche innerhalb des Hohlraumes (11) aufweist, und
das gasdurchlässige zweite Trägermaterial (3) eine Vielzahl von erhabenen Vorsprüngen (4) auf der Oberfläche außerhalb des Hohlraumes (11) und in unmittelbarer Nähe zum Körper des Trägers aufweist.

2. Kleidungsstück nach Anspruch 1, bei dem das erste Trägermaterial ein Laminat aufweist.

3. Kleidungsstück nach Anspruch 1, bei dem das zweite Trägermaterial ein Laminat aufweist.

4. Kleidungsstück nach Anspruch 1, bei dem das erste und zweite Trägermaterial beide ein Laminat aufweisen.

5. Kleidungsstück nach Anspruch 1, bei dem das erste und zweite Trägermaterial wasserdampfdurchlässig sind.

6. Kleidungsstück nach Anspruch 2, bei dem das erste und zweite Trägermaterial ein wasserdampfdurchlässiges Laminat sind.

7. Kleidungsstück nach Anspruch 1, bei dem das zweite Trägermaterial Perforationen aufweist.

8. Kleidungsstück nach Anspruch 1, bei dem das zweite Trägermaterial eine mikroporöse Membran aufweist.

9. Kleidungsstück nach Anspruch 1, bei dem das zweite Trägermaterial ein Gewebe aufweist.

10. Kleidungsstück nach Anspruch 1, bei dem das zweite Trägermaterial einen textilen Vliesstoff aufweist.

11. Kleidungsstück nach Anspruch 1, bei dem das erste und zweite Trägermaterial einen Verdunstungswiderstandswert von weniger als 20 m² Pa W⁻¹ aufweisen.

12. Kleidungsstück nach Anspruch 1, bei dem das erste und zweite Trägermaterial einen Verdunstungswiderstandswert von weniger als 15 m² Pa W⁻¹ aufweisen.

13. Kleidungsstück nach Anspruch 1, bei dem das zweite Trägermaterial eine Luftdurchlässigkeit von zwischen 10 und 100 Liter/m²/sec. bei einem Druck von 100 Pa aufweist.

14. Kleidungsstück nach Anspruch 1, bei dem das Gas über den Körper des Trägers mit einer Geschwindigkeit von zwischen 1 und 10 Liter/Sekunde strömt.

15. Kleidungsstück nach Anspruch 1, bei dem das Gas über den Körper des Trägers mit einer Geschwindigkeit von zwischen 4 und 8 Liter/Sekunde strömt.

16. Kleidungsstück nach Anspruch 1, bei dem die erhabenen Vorsprünge in der Form von diskreten Elementen vorliegen.

17. Kleidungsstück nach Anspruch 1, bei dem die erhabenen Vorsprünge ein thennoplastisches Polymer aufweisen.

18. Kleidungsstück nach Anspruch 1, bei dem die erhabenen Vorsprünge ein duroplastisches Polymer aufweisen.

19. Kleidungsstück nach Anspruch 1, bei dem die erhabenen Vorsprünge auf dem zweiten Trägermaterial eine Höhe von zwischen 1 mm und 20 mm aufweisen.

20. Kleidungsstück nach Anspruch 1, bei dem die erhabenen Vorsprünge auf dem zweiten Trägermaterial eine Höhe von zwischen 2 mm und 4 mm aufweisen.

21. Kleidungsstück nach Anspruch 16, bei dem der Abstand zwischen den diskreten Elementen zwischen 5 mm und 25 mm beträgt.

22. Kleidungsstück nach Anspruch 20, bei dem die erhabenen Vorsprünge diskrete Elemente sind und der Abstand zwischen den diskreten Elementen zwischen 5 mm und 25 mm beträgt.

23. Kleidungsstück nach Anspruch 1, bei dem die erhabenen Vorsprünge innerhalb des Hohlraumes auf dem ersten Trägermaterial angeordnet sind.

24. Kleidungsstück nach Anspruch 1, bei dem die erhabenen Vorsprünge innerhalb des Hohlraumes auf dem zweiten Trägermaterial angeordnet sind.

25. Kleidungsstück nach Anspruch 1, bei dem das erste Trägermaterial PTFE aufweist.

26. Kleidungsstück nach Anspruch 1, bei dem das zweite Trägermaterial PTFE aufweist.

27. Kleidungsstück nach Anspruch 25, bei dem das PTFE expandiertes PTFE aufweist.

28. Kleidungsstück nach Anspruch 26, bei dem das PTFE expandiertes PTFE aufweist.

29. Kleidungsstück nach Anspruch 1, bei dem das erste und zweite Trägermaterial elastisch sind.

30. Kleidungsstück nach Anspruch 1, bei dem das gasdurchlässige zweite Trägermaterial formanpassungsfähig ist.

31. Kleidungsstück nach Anspruch 1, bei dem ein Trägermaterial, das eine Vielzahl von erhabenen Vorsprüngen auf einer Oberfläche aufweist, eine maximale Formanpassungsfähigkeitskraft von weniger als oder gleich 0,03 kg aufweist.

32. Kleidungsstück nach Anspruch 1, bei dem das gasdurchlässige zweite Trägermaterial, das eine Vielzahl von erhabenen Vorsprüngen auf einer Oberfläche aufweist, eine maximale Formanpassungsfähigkeitskraft von weniger als oder gleich 0,03 kg aufweist.

33. Kleidungsstück nach Anspruch 1, bei dem das gasdurchlässige zweite Trägermaterial, das eine Vielzahl von erhabenen Vorsprüngen auf einer Oberfläche aufweist, eine maximale Formanpassungsfähigkeitskraft von weniger als oder gleich 0,02 kg aufweist.

34. Kleidungsstück nach Anspruch 1, bei dem das gasdurchlässige zweite Trägermaterial, das eine Vielzahl von erhabenen Vorsprüngen auf einer Oberfläche aufweist, eine maximale Fonnanpassungsfähigkeitskraft von weniger als oder gleich 0,01 kg aufweist.

35. Kleidungsstück nach Anspruch 1, bei dem der Gasstrom in den Hohlraum hinein Umgebungsluft aufweist.

36. Kleidungsstück nach Anspruch 1, bei dem das Kleidungsstück nicht angebunden ist.

37. Kleidungsstück nach Anspruch 1, bei dem das Gas, das in den Hohlraum strömt, gefiltert oder anderweitig behandelt wird, um unerwünschte Komponenten zu entfernen.

38. Kleidungsstück nach Anspruch 1, bei dem der Hohlraum eine Gasverteilungsleitung aufweist.

39. Kleidungsstück nach Anspruch 1, das nichtentflammbare Materialien aufweist.

40. Kleidungsstück nach Anspruch 1, das nichtschmelzende Materialien aufweist.

41. System für das Abkühlen des Körpers eines Trägers, das aufweist:
ein Kleidungsstück (1), das ein im Wesentlichen gasundurchlässiges erstes Trägermaterial (2) und ein gasdurchlässiges zweites Trägermaterial (3) aufweist, das um den Umfang der Trägermaterialien herum befestigt ist, wodurch ein Hohlraum (11) dazwischen gebildet wird,
wobei der Hohlraum (11) so angepasst ist, dass er mit einer Gaszuführung verbunden wird, so dass das Gas in den Hohlraum (11) strömt und aus dem Hohlraum (11) durch das gasdurchlässige zweite Trägermaterial (3) austritt;
**dadurch gekennzeichnet, dass**
mindestens eines von erstem und zweitem Trägermaterial (2, 3) eine Vielzahl von erhabenen Vorsprüngen (12) auf einer Oberfläche innerhalb des Hohlraumes (11) aufweist, und
mindestens ein zusätzliches Trägermaterial, das zwischen dem Körper des Trägers und dem gasdurchlässigen zweiten Trägermaterial (3) des Kleidungsstückes (1) angeordnet ist, wobei das mindestens eine zusätzliche Trägermaterial eine Vielzahl von erhabenen Vorsprüngen auf einer Oberfläche des Trägermaterials aufweist.

42. System nach Anspruch 41, bei dem das mindestens eine zusätzliche Trägermaterial am Kleidungsstück befestigt ist.

43. System nach Anspruch 41, bei dem das mindestens eine zusätzliche Trägermaterial am Kleidungsstück lösbar befestigt ist.

44. System nach Anspruch 41, bei dem das mindestens eine zusätzliche Trägermaterial und das Kleidungsstück getrennt sind.

45. System nach Anspruch 41, bei dem das erste Trägermaterial ein Laminat aufweist.

46. System nach Anspruch 41, bei dem das zweite Trägermaterial ein Laminat aufweist.

47. System nach Anspruch 41, bei dem das mindestens eine zusätzliche Trägermaterial ein Laminat aufweist.

48. System nach Anspruch 41, bei dem das erste und zweite Trägermaterial beide ein Laminat aufweisen.

49. System nach Anspruch 41, bei dem das erste Trägermaterial wasserdampfdurchlässig ist.

50. System nach Anspruch 41, bei dem das zweite Trägermaterial wasserdampfdurchlässig ist.

51. System nach Anspruch 41, bei dem das zweite Trägermaterial Perforationen aufweist.

52. System nach Anspruch 41, bei dem das zweite Trägermaterial eine mikroporöse Membran aufweist.

53. System nach Anspruch 41, bei dem das mindestens eine zusätzliche Trägermaterial ein Gestrick, Gewebe oder einen textilen Vliesstoff aufweist.

54. System nach Anspruch 41, bei dem das mindestens eine zusätzliche Trägermaterial wasserdampfdurchlässig ist.

55. System nach Anspruch 54, bei dem das mindestens eine zusätzliche Trägermaterial gasdurchlässig ist.

56. System nach Anspruch 55, bei dem das mindestens eine zusätzliche Trägermaterial Perforationen aufweist.

57. System nach Anspruch 55, bei dem das mindestens eine zusätzliche Trägermaterial erhabene Vorsprünge aufweist, die in Richtung des Körpers eines Trägers ausgerichtet sind.

58. System nach Anspruch 54, bei dem das mindestens eine zusätzliche Trägermaterial erhabene Vorsprünge aufweist, die weg vom Körper eines Trägers ausgerichtet sind.

59. System nach Anspruch 58, bei dem das mindestens eine zusätzliche Trägermaterial gasdurchlässig ist.

60. System nach Anspruch 41, bei dem das mindestens eine zusätzliche Trägermaterial Unterkleidung aufweist.

61. System nach Anspruch 41, bei dem das mindestens eine zusätzliche Trägermaterial ein T-Shirt aufweist.

62. System nach Anspruch 41, bei dem das mindestens eine zusätzliche Trägermaterial einen Verdunstungswiderstandswert von weniger als 20 m² Pa W⁻¹ aufweist.

63. System nach Anspruch 41, bei dem das mindestens eine zusätzliche Trägermaterial einen Verdunstungswiderstandswert von weniger als 15 m² Pa W⁻¹ aufweist.

64. System nach Anspruch 55, bei dem das mindestens eine zusätzliche Trägermaterial eine Luftdurchlässigkeit von zwischen 10 und 100 Liter/m²/sec. bei einem Druck von 100 Pa aufweist.

65. System nach Anspruch 47, bei dem der Gasstrom über den Körper des Trägers von zwischen 1 und 10 l/sec. beträgt.

66. System nach Anspruch 41, bei dem der Gasstrom über den Körper des Trägers zwischen 4 und 8 l/sec. beträgt.

67. System nach Anspruch 41, bei dem die erhabenen Vorsprünge in der Form von diskreten Elementen vorliegen.

68. System nach Anspruch 41, bei dem die erhabenen Vorsprünge ein Polymer aufweisen.

69. System nach Anspruch 41, bei dem die erhabenen Vorsprünge ein thermoplastisches Polymer aufweisen.

70. System nach Anspruch 41, bei dem die erhabenen Vorsprünge ein duroplastisches Polymer aufweisen.

71. System nach Anspruch 41, bei dem die erhabenen Vorsprünge eine Höhe von zwischen 1 mm und 20 mm aufweisen.

72. System nach Anspruch 41, bei dem die erhabenen Vorsprünge auf dem mindestens einem zusätzlichen Trägermaterial eine Höhe von zwischen 2 mm und 4 mm aufweisen.

73. System nach Anspruch 41, bei dem der Abstand zwischen den erhabenen Vorsprüngen zwischen 5 mm und 25 mm beträgt.

74. System nach Anspruch 72, bei dem der Abstand zwischen den erhabenen Vorsprüngen zwischen 5 mm und 25 mm beträgt.

75. System nach Anspruch 41, bei dem das erste Trägermaterial PTFE aufweist.

76. System nach Anspruch 41, bei dem das zweite Trägermaterial PTFE aufweist.

77. System nach Anspruch 75, bei dem das PTFE expandiertes PTFE aufweist.

78. System nach Anspruch 76, bei dem das PTFE expandiertes PTFE aufweist.

79. System nach Anspruch 41, bei dem das erste und zweite Trägermaterial elastisch sind.

80. System nach Anspruch 41, bei dem das mindestens eine zusätzliche Trägermaterial forinanpassungsfähig ist.

81. System nach Anspruch 41, bei dem ein Trägermaterial, das eine Vielzahl von erhabenen Vorsprüngen aufweist, eine maximale Formanpassungsfähigkeitskraft von weniger als oder gleich 0,03 kg aufweist.

82. System nach Anspruch 41, bei dem das gasdurchlässige zweite Trägermaterial eine Vielzahl von erhabenen Vorsprüngen und eine maximale Formanpassungsfähigkeitskraft von weniger als oder gleich 0,03 kg aufweist.

83. System nach Anspruch 41, bei dem das gasdurchlässige zweite Trägermaterial eine Vielzahl von erhabenen Vorsprüngen aufweist und eine maximale Formanpassungsfähigkeitskraft von weniger als oder gleich 0,02 kg aufweist.

84. System nach Anspruch 41, bei dem das gasdurchlässige zweite Trägermaterial eine Vielzahl von erhabenen Vorsprüngen und eine maximale Formanpassungsfähigkeitskraft von weniger als oder gleich 0,01 kg aufweist.

85. System nach Anspruch 41, bei dem der Gasstrom in den Hohlraum hinein Umgebungsluft aufweist.

86. System nach Anspruch 41, bei dem das Gas, das in den Hohlraum strömt, gefiltert oder anderweitig behandelt wird, um unerwünschte Komponenten zu entfernen.

87. System nach Anspruch 41, bei dem der Hohlraum eine Gasverteilungsleitung aufweist.

88. System nach Anspruch 41, das nichtentflammbare Materialien aufweist.

89. System nach Anspruch 41, das nichtschmelzende Materialien aufweist.

90. System nach Anspruch 41, bei dem das System nicht angebunden ist.

## Revendications

1. Vêtement (1) permettant de refroidir le corps d'un utilisateur comprenant:
un premier substrat (2) pratiquement imperméable aux gaz et un second substrat (3) perméable aux gaz attachés ensemble le long des périphéries des substrats, formant une cavité (11) entre ceux-ci, où la cavité (11) est conçue pour être reliée à une alimentation en gaz de sorte que le gaz circule dans la cavité (11) et sort de la cavité (11) par le second substrat (3) perméable aux gaz; **caractérisé en ce que**
au moins un des premier et second substrats (2, 3) comprend une pluralité de protubérances en relief (12) sur une surface à l'intérieur de la cavité (11), et
le second substrat (3) perméable aux gaz comprend une pluralité de protubérances en relief (4) sur la surface extérieure à la cavité (11) et proche du corps de l'utilisateur.

2. Vêtement selon la revendication 1 dans lequel le premier substrat comprend un stratifié.

3. Vêtement selon la revendication 1 dans lequel le second substrat comprend un stratifié.

4. Vêtement selon la revendication 1 dans lequel les premier et second substrats comprennent chacun un stratifié.

5. Vêtement selon la revendication 1 dans lequel les premier et second substrats sont perméables à la vapeur d'eau.

6. Vêtement selon la revendication 2 dans lequel les premier et second substrats sont des stratifiés perméables à la vapeur d'eau.

7. Vêtement selon la revendication 1 dans lequel le second substrat comprend des perforations.

8. Vêtement selon la revendication 1 dans lequel le second substrat comprend une membrane microporeuse.

9. Vêtement selon la revendication 1 dans lequel le second substrat comprend un textile tissé.

10. Vêtement selon la revendication 1 dans lequel le second substrat comprend un textile non tissé.

11. Vêtement selon la revendication 1 dans lequel les premier et second substrats ont une valeur de résistance à l'évaporation inférieure à 20 m².Pa.W⁻¹.

12. Vêtement selon la revendication 1 dans lequel les premier et second substrats ont une valeur de résistance à l'évaporation inférieure à 15 m².Pa.W⁻¹.

13. Vêtement selon la revendication 1 dans lequel le second substrat a une perméabilité à l'air comprise entre 10 et 100 litres/m²/seconde à une pression de 100 Pa.

14. Vêtement selon la revendication 1 dans lequel le gaz circule sur le corps de l'utilisateur à une vitesse comprise entre 1 et 10 litres/seconde.

15. Vêtement selon la revendication 1 dans lequel le gaz circule sur le corps de l'utilisateur à une vitesse comprise entre 4 et 8 litres/seconde.

16. Vêtement selon la revendication 1 dans lequel les protubérances en relief se présentent sous la forme d'éléments discrets.

17. Vêtement selon la revendication 1 dans lequel les protubérances en relief comprennent un polymère thermoplastique.

18. Vêtement selon la revendication 1 dans lequel les protubérances en relief comprennent un polymère thermodurcissable.

19. Vêtement selon la revendication 1 dans lequel les protubérances en relief sur le second substrat ont une hauteur comprise entre 1 et 20 mm.

20. Vêtement selon la revendication 1 dans lequel les protubérances en relief sur le second substrat ont une hauteur comprise entre 2 et 4 mm.

21. Vêtement selon la revendication 16 dans lequel la distance séparant les éléments discrets est comprise entre 5 mm et 25 mm.

22. Vêtement selon la revendication 20 dans lequel les protubérances en relief sont des éléments discrets et la distance séparant les éléments discrets est comprise entre 5 mm et 25 mm.

23. Vêtement selon la revendication 1 dans lequel les protubérances en relief à l'intérieur de la cavité sont situées sur le premier substrat.

24. Vêtement selon la revendication 1 dans lequel les protubérances en relief à l'intérieur de la cavité sont situées sur le second substrat.

25. Vêtement selon la revendication 1 dans lequel le premier substrat comprend du PTFE.

26. Vêtement selon la revendication 1 dans lequel le second substrat comprend du PTFE.

27. Vêtement selon la revendication 25 dans lequel le PTFE comprend du PTFE expansé.

28. Vêtement selon la revendication 26 dans lequel le PTFE comprend du PTFE expansé.

29. Vêtement selon la revendication 1 dans lequel les premier et second substrats sont souples.

30. Vêtement selon la revendication 1 dans lequel le second substrat perméable aux gaz est conformable.

31. Vêtement selon la revendication 1 dans lequel un substrat comprenant une pluralité de protubérances en relief sur une surface possède une force maximale de conformabilité inférieure ou égale à 0,03 kg.

32. Vêtement selon la revendication 1 dans lequel le second substrat perméable aux gaz comprenant une pluralité de protubérances en relief sur une surface possède une force maximale de conformabilité inférieure ou égale à 0,03 kg.

33. Vêtement selon la revendication 1 dans lequel le second substrat perméable aux gaz comprenant une pluralité de protubérances en relief sur une surface possède une force maximale de conformabilité inférieure ou égale à 0,02 kg.

34. Vêtement selon la revendication 1 dans lequel le second substrat perméable aux gaz comprenant une pluralité de protubérances en relief sur une surface possède une force maximale de conformabilité inférieure ou égale à 0,01 kg.

35. Vêtement selon la revendication 1 dans lequel le flux de gaz dans ladite cavité comprend de l'air ambiant.

36. Vêtement selon la revendication 1 dans lequel le vêtement est non attaché.

37. Vêtement selon la revendication 1 dans lequel le gaz circulant dans la cavité est filtré ou traité d'une autre manière pour éliminer les composants indésirables.

38. Vêtement selon la revendication 1 dans lequel ladite cavité comprend une rampe de distribution de gaz.

39. Vêtement selon la revendication 1 qui comprend des matériaux non inflammables.

40. Vêtement selon la revendication 1 qui comprend des matériaux ne fondant pas.

41. Système permettant de refroidir le corps d'un utilisateur comprenant
un vêtement (1) comprenant un premier substrat (2) pratiquement imperméable aux gaz et un second substrat (3) perméable aux gaz attachés ensemble le long des périphéries des substrats, formant une cavité (11) entre ceux-ci, où la cavité (11) est conçue pour être reliée à une alimentation en gaz de sorte que le gaz circule dans la cavité (11) et sort de la cavité (11) par le second substrat (3) perméable aux gaz; **caractérisé en ce que**
au moins un des premier et second substrats (2, 3) comprend une pluralité de protubérances en relief (12) sur une surface à l'intérieur de la cavité (11), et
au moins un substrat supplémentaire est intercalé entre le corps de l'utilisateur et le second substrat (3) perméable aux gaz du vêtement (1), ledit au moins un substrat supplémentaire comprenant une pluralité de protubérances en relief (4) sur une surface du substrat.

42. Système selon la revendication 41 dans lequel ledit au moins un substrat supplémentaire est fixé au vêtement.

43. Système selon la revendication 41 dans lequel ledit au moins un substrat supplémentaire est fixé au vêtement de manière à pouvoir s'en détacher.

44. Système selon la revendication 41 dans lequel ledit au moins un substrat supplémentaire et le vêtement sont séparés.

45. Système selon la revendication 41 dans lequel le premier substrat comprend un stratifié.

46. Système selon la revendication 41 dans lequel le second substrat comprend un stratifié.

47. Système selon la revendication 41 dans lequel ledit au moins un substrat supplémentaire comprend un stratifié.

48. Système selon la revendication 41 dans lequel les premier et second substrats comprennent chacun un stratifié.

49. Système selon la revendication 41 dans lequel le premier substrat est perméable à la vapeur d'eau.

50. Système selon la revendication 41 dans lequel le second substrat est perméable à la vapeur d'eau.

51. Système selon la revendication 41 dans lequel le second substrat comprend des perforations.

52. Système selon la revendication 41 dans lequel le second substrat comprend une membrane microporeuse.

53. Système selon la revendication 41 dans lequel ledit au moins un substrat supplémentaire comprend un textile tricoté, tissé ou non tissé.

54. Système selon la revendication 41 dans lequel ledit au moins un substrat supplémentaire est perméable à la vapeur d'eau.

55. Système selon la revendication 54 dans lequel ledit au moins un substrat supplémentaire est perméable aux gaz.

56. Système selon la revendication 55 dans lequel ledit au moins un substrat supplémentaire comprend des perforations.

57. Système selon la revendication 55 dans lequel ledit au moins un substrat supplémentaire comprend des protubérances en relief orientées vers le corps d'un utilisateur.

58. Système selon la revendication 54 dans lequel ledit au moins un substrat supplémentaire comprend des protubérances en relief orientées à distance du corps d'un utilisateur.

59. Système selon la revendication 58 dans lequel ledit au moins un substrat supplémentaire est perméable aux gaz.

60. Système selon la revendication 41 dans lequel ledit au moins un substrat supplémentaire comprend un sous-vêtement.

61. Système selon la revendication 41 dans lequel ledit au moins un substrat supplémentaire comprend un tee-shirt.

62. Système selon la revendication 41 dans lequel ledit au moins un substrat supplémentaire a une valeur de résistance à l'évaporation inférieure à 20 m².Pa.W⁻¹.

63. Système selon la revendication 41 dans lequel ledit au moins un substrat supplémentaire a une valeur de résistance à l'évaporation inférieure à 15 m².Pa.W⁻¹.

64. Système selon la revendication 55 dans lequel ledit au moins un substrat supplémentaire a une perméabilité à l'air comprise entre 10 et 100 litres/m²/seconde à une pression de 100 Pa.

65. Système selon la revendication 41 dans lequel la vitesse de circulation du gaz sur le corps de l'utilisateur est comprise entre 1 et 10 litres/seconde.

66. Système selon la revendication 41 dans lequel la vitesse de circulation du gaz sur le corps de l'utilisateur est comprise entre 4 et 8 litres/seconde.

67. Système selon la revendication 41 dans lequel les protubérances en relief se présentent sous la forme d'éléments discrets.

68. Système selon la revendication 41 dans lequel les protubérances en relief comprennent un polymère.

69. Système selon la revendication 41 dans lequel les protubérances en relief comprennent un polymère thermoplastique.

70. Système selon la revendication 41 dans lequel les protubérances en relief comprennent un polymère thermodurcissable.

71. Système selon la revendication 41 dans lequel les protubérances en relief ont une hauteur comprise entre 1 mm et 20 mm.

72. Système selon la revendication 41 dans lequel les protubérances en relief sur ledit au moins un substrat supplémentaire ont une hauteur comprise entre 2 mm et 4 mm.

73. Système selon la revendication 41 dans lequel 1a distance séparant les protubérances en relief est comprise entre 5 mm et 25 mm.

74. Système selon la revendication 72 dans lequel la distance séparant les protubérances en relief est comprise entre 5 mm et 25 mm.

75. Système selon la revendication 41 dans lequel le premier substrat comprend du PTFE.

76. Système selon la revendication 41 dans lequel le second substrat comprend du PTFE.

77. Système selon la revendication 75 dans lequel le PTFE comprend du PTFE expansé.

78. Système selon la revendication 76 dans lequel le PTFE comprend du PTFE expansé.

79. Système selon la revendication 41 dans lequel les premier et second substrats sont souples.

80. Système selon la revendication 41 dans lequel ledit au moins un substrat supplémentaire est conformable.

81. Système selon la revendication 41 dans lequel un substrat comprenant une pluralité de protubérances en relief possède une force maximale de conformabilité inférieure ou égale à 0,03 kg.

82. Système selon la revendication 41 dans lequel le second substrat perméable aux gaz comprend une pluralité de protubérances en relief et possède une force maximale de conformabilité inférieure ou égale à 0,03 kg.

83. Système selon la revendication 41 dans lequel le second substrat perméable aux gaz comprend une pluralité de protubérances en relief et possède une force maximale de conformabilité inférieure ou égale à 0,02 kg.

84. Système selon la revendication 41 dans lequel le second substrat perméable aux gaz comprend une pluralité de protubérances en relief et possède une force maximale de conformabilité inférieure ou égale à 0,01 kg.

85. Système selon la revendication 41 dans lequel le flux de gaz dans ladite cavité comprend de l'air ambiant.

86. Système selon la revendication 41 dans lequel le gaz circulant dans la cavité est filtré ou traité d'une autre manière pour éliminer les composants indésirables.

87. Système selon la revendication 41 dans lequel ladite cavité comprend une rampe de distribution de gaz.

88. Système selon la revendication 41 qui comprend des matériaux non inflammables.

89. Système selon la revendication 41 qui comprend des matériaux ne fondant pas.

90. Système selon la revendication 41 dans lequel le système est non attaché.
